# EUROPEAN PATENT SPECIFICATION

(11) **EP 0 435 293 B1**
(45) Date of publication and mention of the grant of the patent: **20.03.1996**
(21) Application number: 90125598.4
(22) Date of filing: 27.12.1990
(51) Int. Cl.: C12P 41/00, C12P 7/62, C12P 17/04

(54) **Methods of producing optically active hydroxyesters**
Verfahren zur Herstellung von optisch-aktiven Hydroxyestern
Procédé de préparation d'esters hydroxylés optiquement actives

(30) Priority: 27.12.1989 JP 341578/89; 27.12.1989 JP 341579/89; 13.03.1990 JP 63665/90; 03.04.1990 JP 89669/90
(43) Date of publication of application: 03.07.1991
(73) Proprietor: Nitto Denko Co. Ltd., Ibaraki City, Osaka 567 (JP)
(72) Inventor: Fukusaki, Eiichiro, c/o Nitto Denko Co.Ltd., Ibaraki City, Osaka 567 (JP); Senda, Shuji, c/o Nitto Denko Co.Ltd., Ibaraki City, Osaka 567 (JP); Nakazono, Yutaka, c/o Nitto Denko Co.Ltd., Ibaraki City, Osaka 567 (JP); Omata, Tetsuo, c/o Nitto Denko Co.Ltd., Ibaraki City, Osaka 567 (JP); Hibino, Ken, c/o Nitto Denko Co.Ltd., Ibaraki City, Osaka 567 (JP); Ota, Hiromichi, Setagayaku, Tokyo 157 (JP); Sugai, Takeshi, Kawasaki City, Kanagawa 211 (JP)
(74) Representative: Brauns, Hans-Adolf, Dr. rer. nat.

(56) References cited:
- JP-A-63 036 339
- PATENT ABSTRACTS OF JAPAN, vol. 13, no. 315 (C-619)(3663), 18 July 1989/
- PATENT ABSTRACTS OF JAPAN, vol. 9, no. 1 (C-259)(1724), 05 January 1985/
- CHEMICAL ABSTRACTS, vol. 110, no. 13, 27 March 1989, Columbus, OH (US); H. AKITA et al., p. 560, no. 113228/
- TETRAHEDRON LETTERS, vol. 28, no. 33, 1987, Oxford (GB); A.L. GUTMAN et al., pp. 3861-3864/
- TETRAHEDRON LETTERS, vol. 28, no. 7, 1987, Oxford (GB); M. ATUSHI et al., pp. 805-808/
- TETRAHEDRON LETTERS, vol. 29, no. 16, 1988, Oxford (GB); L. BLANCO et al., pp. 1915-1918/
- SYNTHESIS, no. 12, December 1990, Stuttgart (DE); T. SUGAI et al., pp. 1112- 1114/

## Description

This invention relates to a method for producing optically active hydroxyesters. More specifically, the invention provides a method for efficiently, safely, and selectively producing and purifying optically active substances with a high degree of purity at ordinary temperatures.

The optically active hydroxyesters provided by the method of this invention are useful as intermediates for synthesizing various medicines, agricultural chemicals, and biologically active substances. They are however especially useful for synthesizing a pheromone of the noxious insect, *Popillia japonica* Newman.

### BACKGROUND OF THE INVENTION

Optically active hydroxyesters have been noted as important intermediates for synthesizing various medicines, agricultural chemicals, and biologically active substances.

Especially an optically active (R)-4-hydroxy-tetradecynoate, an example of the (R)-4-hydroxyesters of formula 1, and optically active (R)-5-(1-decynyl)-oxacyclopentan-2-one, an example of the (R)-five-membered ring lactones of formula 5 , are important intermedeates for synthesizing a pheromone of the noxious insect, *Popillia japonica* Newman (see Shuji Senda and Kenji Mori, Agrical. Biol. Chem., 47 (11) 2595 to 2598 (1983)).

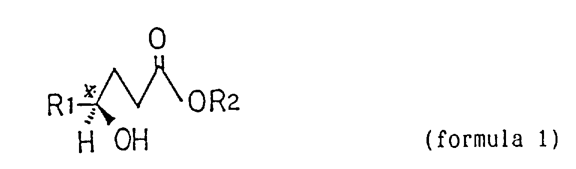

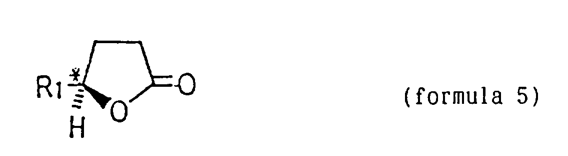

In the above formulae, R₁ denotes -C ≡ C-R₃ or -C=C-R₃; R₂ and R₃ denote straight chain or branched alkyl groups containing 1 to 10 carbon atoms; and * denotes asymmetric carbon atoms.

Optically active (R)-4-hydroxyesters of formula 1 and optically active (R)-five-membered ring lactones of formula 5 are interconvertible.

Optically active (R)-4-hydroxyesters are lactonized to optically active (R)-five-membered ring lactones in the usual way.

On the other hand, optically active (R)-five-membered ring lactones are hydrolyzed and esterificated to optically active (R)-4-hydroxyesters.

The activity of the pheromone of *Popillia jaonica* Newman, (R,Z)-(-)-5-(1-decenyl)-dihydro-2-(3H)-furanone, is drastically reduced in the presence of its (S,Z)-isomer.

It is said that the presence of 0.5% of the (S,Z)-isomer lowers the activity of the pheromone to 60%, 2% of the (S,Z)-isomer to one third, and 6% of the (S,Z)-isomer to zero.

It is accordingly very important to produce the intermediates, (R)-4-hydroxy-5-tetra-decynoates and (R)-5-(1-decinyl)-oxacyclopentan-2-one, in a high state of purity.

However, no efficient synthesis and purifying process has been provided so far.

### PRIOR ART

Methods of producing optically active (R)-4-hydroxy-5-tetra-decynoates (one of (R)-4-hydroxyesters) have been proposed.

The inventors have disclosed the chemical synthesis and microbial synthesis of optically active (R)-4-hydroxy-5-tetra-decynoates from 4-oxo-5-tetradecynoates (Japanese Published Unexamined Patent Applications No. Sho 59-157,055, and No. Hei 1-101,889, respectively).

The production of optically active (R)-five-membered ring lactones has also been disclosed; they are obtained by lactonzing optically active (R)-4-hydroxy-5-tetradecynoates.

### PROBLEMS OF THE PRIOR ART

Although the chemical synthesis (Japanese Published Unexamined Patent Application No. Sho 59-157,055) gives (R)-4-hydroxy-5-tetradecynoates in a state of high purity, it has some disadvantages and so is not practical.

These disadvantages include;
(1) The method requires expensive asymmetric reducing agents;
(2) The temperature should be controlled to - 100 °C and
(3) It requires expensive and dangerous reagents such as lithium aluminum hydride.

The microbial synthesis (Japanese Published Unexamined Patent Application No. Hei 1-101,889) does not always give so (R)-4-hydroxy-5-tetradecynoates in high yield and so is not practical.

Accordingly, the synthesis of optically active (R)-five-membered ring lactones from (R)-4-hydroxy-5-tetradecynoates does not provide a high purity product and so is not practical.

Furthermore, no practical methods have been proposed for purifying the reaction product including (R)-five-membered ring lactones of insufficient optically purity so as to obtain optically active pure (R)-five-membered ring lactones.

### DETAILED DESCRIPTION OF THE INVENTION

The inventors have devised a method (A) for producing optically active hydroxyesters as a result of their serious study.

The method (A) for producing optically active hydroxyesters provided by the present invention uses an impure optically active hydroxy-ester that is a mixture of the optically active (R)-hydroxyester (shown by formula 1) and the optically active (S)-hydroxyester (shown by formula 2).

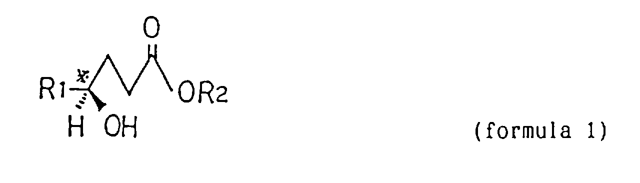

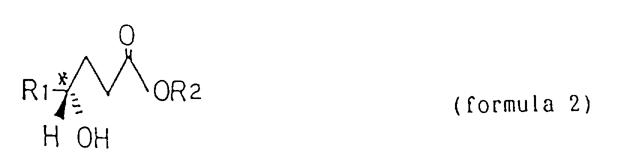

In the above formulae, R₁ denotes -C≡C-R₃ or -C=C-R₃; R₂ and R₃ denote straight chain or branched alkyl groups containing 1 to 10 carbon atoms; and * denotes asymmetric carbon atoms.

Any mixture containing optically active (R)-hydroxy ester (formula 1) and optically active (S)-hydroxyester (formula 2) can be used as a starting material as the above mixture, therefore for example a racemic mixture of the hydroxyester (formula 6) is favourably used 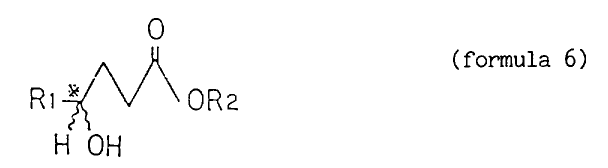 wherein R₁ denotes -C≡C-R₃ or -C=C-R₃; R₂ and R₃ denote straight chain or branched alkyl groups containing 1 to 10 carbon atoms; and * denotes an asymmetric carbon atom.

This racemic hydroxyester (formula 6) is easily gained or produced.

An example for synthesizing a 4-hydroxy-5-tetradecynoic acid derivative is to take a 4-oxo-5-tetradecynoic acid derivative as a starting material obtained by a well known manner (Agric.Biol.47(11),2595∼2598, 1983), and dissolve this in a solvent such as methanol. It is then reduced with sodium boron hydride.

Examples of the racemic hydroxyester (formula 6) are methyl 4-hydroxy-5-tetradecynoate, ethyl 4-hydroxy-5-tetradecynoate, ethyl 4-hydroxy-5-tetradecynoate, ethyl 4-hydroxy-5-tetradecenoate, methyl 4-hydroxy-5-tetra-decenoate, methyl 4-hydroxy-5-tridecynoate, or methyl 4-hydroxy-5-tetra-dodecynoate.

Especially, methyl 4-hydroxy-5-tetradecynoate is important as an intermediate for synthesizing a sex pheromon of *Popillia japonica* Newman, a noxious insect for grass.

In this invention, the racemic hydroxyester (formula 6) is reacted with a carboxylic acid or a carboxylic anhydride in the presence of a hydrolase in an organic solvent so as to selectively acylate the optically active (R)-hydroxyester (formula 1) to the optically active (R)-diester (shown by formula 3). 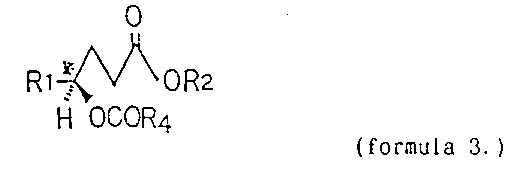 wherein R₁ denotes -C≡C-R₃ or -C=C-R₃; R₂ and R₃ denote straight chain or branched alkyl groups containing 1 to 10 carbon atoms; R4 denotes an alkyl group, an aralkyl group, or a haloalkyl group containing 1 to 12 carbon atoms and optionally terminated with a carboxyl group; and * denotes an asymmetric carbon atom.

In this reaction, the enzyme to be used is a lipase such as a lipase derived from the pancreas of a pig, yeast, mold, bacteria, or an esterase such as an esterase derived from the liver of a pig, or a cholesterolesterase.

Either purified or crude enzymes can be used, and the state of enzymes is not restricted. The enzyme may favourably be used in any state of powder, grain or dried biomass of microorganism (treated biomass and paused biomass) containing the enzyme etc.

These enzymes can be used as they are, or immobilized on a carrier.

The organic solvent favourably used in this reaction can be any nonaqueous system solvent, for example an acyclic hydrocarbon solvent such as n-hexane, n-heptane, n-octane, isobutane, isopentane, isooctane, and a cyclic hydrocarbon solvent such as cyclopentane, cyclohexane, and a halogenated hydrocarbon solvent such as dichloromethane, trichloromethane, and an aromatic hydrocarbon solvent such as benzene, toluene, xylene, and an ether group solvent such as diethylether, diisopropylether, n-butylether, tetrahydrofuran, tetrahydropyran and carbon tetrachloride.

The carboxylic acid used in this reaction can be any carboxylic acid which can be used as a substrate by a hydrolase, and more favourably used carboxylic acids have a carbon number in the range of 2 to 10.

Examples of carboxylic acids are acetic acid, propionic acid, butyric acid, valeric acid or capronic acid.

The carboxylic anhydride in this reaction can be any carboxylic anhydride which can be used as a substrate by a hydrolase, and more favourably used are an acylic carboxylic anhydride having a carbon number from 2 to 10, or a cyclic carboxylic anhydride having a carbon number from 4 to 10.

These examples of anhydrides are acylic carboxylic anhydride such as the anhydride of acetic acid, propionic acid, butyric acid, valeric acid, capronic acid, and cyclic carboxylic acid anhydrides such as succinic anhydride, maleic anhydride and glutaric anhydride.

The combination ratio of racemic hydroxyester (formula 6) and acyl group donator (a carboxylic acid or a carboxylic anhydride) is preferably 1: not less than 0.5 by molar ratio.

The reaction temperature in this reaction is preferably within the active temperature range of the enzyme, and ordinarily it is in the range of 10°C to 50°C.

After the asymmetric acylation reaction, the reaction mixture is separated into the optically active (R)-diester (formula 3) and the optically active (S)-hydroxyester (formula 2).

As examples of this separating process, there are extraction by using either an organic solvent being slightly soluble in water, or two solvents of insoluble organic solvent and water as a two layer system, separation by using a column, and distillation.

The optically active (R)-diester (formula 3) obtained in the above manner can be easily converted into the optically active (R)-hydroxyester (formula 1) having the same purity as the diester (formula 3) by hydrolyzing with an alkali such as potassium hydroxide and others, and esterifying with cyanamid and others.

As previously mentioned, methyl 4-hydroxy-5-tetradecynoate is important as an intermediate for synthesizing a sex pheromon of *Papillia japonica* Newman, a noxious insect for grass. This compound can be obtained in optically active form by the method (A) provided by the present invention.

Hereinafter, detailed Examples are described to illustrate the method of this Invention.

### (Example 1.)

Toluene(50ml), racemic methyl 4-hydroxy-5-tetradecynoate (500mg), and n-butyric acid (300mg) were poured into a 100ml Erlenmeyer flask.

In the solution,lipase (500mg) [trade name:Amano-P,maker :Amano Pharmacy] was added, and stirred to react for 16 hours by a magnetic stirrer at 25 °C.

The reaction mixture was filtrated, then the solvent in the obtained filtrate was removed by using a rotary evaporater, thereafter it was concentrated.

The oily substance obtained by the above manner was separated by silica gel chromatography (hexane/ethyl acetate) to be methyl (S)-4-hydroxy-5-tetradecynoate (yield amount of product;300mg,optical purity;60%ee.) and methyl (R)-4-butyryloxy-5-tetradecynoate (yield amount of product;230mg, optical purity;94%ee.).

The optical purity was measured with NMR under Eu(tfc₃). (Note; optical purities in the remaining Examples were measured in the same manner as in Example 1. )

### (Example 2. )

Toluene(50ml),racemic methyl 4-hydroxy-5-tetradecynoate (500mg), and n-capronic acid (300mg) were poured into a 100ml Erlenmeyer flask.

In the solution,lipase(500mg) [trade name:Amano-P,maker :Amano Pharmacy] was added, and stirred to react for 16 hours by a magnetic stirrer at 25 °C.

The reaction mixture was filtrated, then the solvent in the obtained filtrate was removed by using a rotary evaporater, thereafter it was concentrated.

The oily substance obtained by the above manner was separated by silica gel chromatography (hexane/ethyl acetate) to be methyl (S)-4-hydroxy-5-tetradecynoate (yield amount of product;320mg, optical purity;50%ee.) and methyl (R)-4-capryloxy-5-tetradecynoate (yield amount of product;200mg, optical purity;90%ee.).

### (Example 3. )

Toluene(50ml), racemic methyl 4-hydroxy-5-tetradecynoate (500mg), and acetic anhydride (300mg) were poured into a 100ml Erlenmeyer flask.

In the solution,lipase(500mg) [trade name:Amano-P,maker :Amano Pharmacy] was added , and stirred to react for 14 hours by a magnetic stirrer at 25 °C.

The reaction mixture was filtrated, then the solvent in the obtained filtrate was removed by using a rotary evaporater, thereafter it was concentrated.

The oily substance obtained by the above manner was separated by silica gel chromatography (hexane/ethyl acetate) to be methyl (S)-4-hydroxy-5-tetradecynoate (yield amount of product;330mg, optical purity;40%ee.) and methyl (R)-4-acetoxy-5-tetradecynoate (yield amount of product;200mg, optical purity;75%ee.).

### (Example 4. )

Toluene(50ml), racemic metyl 4-hydroxy-5-tetradecynoate (500mg), and butyric anhydride (400mg) were poured into a 100ml Erlenmeyer flask.

In the solution,lipase(500mg)[trade name:Amano-P,maker :Amano Pharmacy] was added, and stirred to react for 14 hours by a magnetic stirrer at 25 °C.

The reaction mixture was filtrated, then the solvent in the obtained filtrate was removed by using a rotary evaporater, thereafter it was concentrated.

The oily substance obtained by the above manner was separated by silica gel chromatography (hexane/ethyl acetate) to be methyl (S)-4-hydroxy-5-tetradecynoate (yield amount of product;360mg, optical purity;38%ee.) and methyl (R)-4-butyryloxy-5-tetradecynoate (yield amount of product;l90mg, optical purity;90%ee.).

### (Example 5. )

Toluene(50ml), racemic methyl 4-hydroxy-5-tetradecynoate (500mg), and succinic anhydride(500mg) were poured into a 100ml Erlenmeyer flask.

In the solution,lipase(500mg) [trade name:Amano-P,maker :Amano Pharmacy] was added , and stirred to react for 14 hours by a magnetic stirrer at 25 °C.

The reaction mixture was filtrated, then the solvent in the obtained filtrate was removed by using a rotary evaporater, thereafter it was concentrated.

The oily substance obtained by the above manner was separated by silica gel chromatography (hexane/ethyl acetate) to be methyl (S)-4-hydroxy-5-tetradecynoate (yield amount of product;250mg, optical purity;70%ee.) and half-ester of succinic acid and 4-position hydroxy group of methyl (R)-4-hydroxy-5-tetradecynoate (yield amount of product;280mg,optical purity;94%ee.).

## Claims

1. A method for producing an optically active hydroxyester comprising the steps of:
(i) adding a carboxylic acid or carboxylic anhydride to a mixture comprising an (R)-hydroxyester of the below formula 1 and an (S)-hydroxyester of the below formula 2 in the presence of a hydrolase in an organic solvent to selectively acylate the optically active (R) -hydroxyester to an optically active (R)-diester of the below formula 3, and
(ii) separating the optically active (S)-hydroxyester from the optically active (R)-diester: 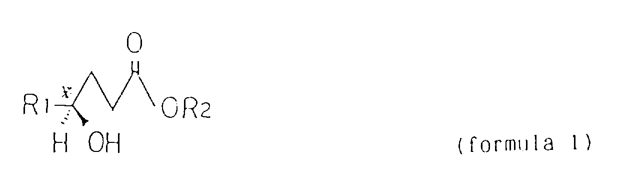 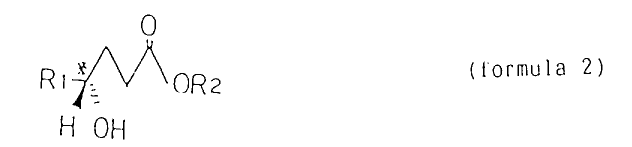 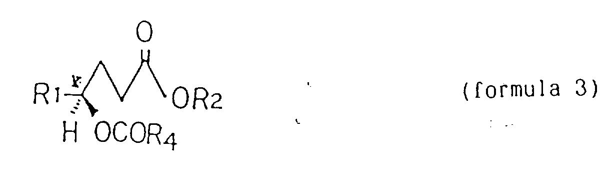
wherein R₁ represents -C≡C-R₃ or -C=C-R₃; and R₂ and R₃ represent a straight chain or branched alkyl group containing 1 to 10 carbon atoms; R₄ represents an alkyl group, an aralkyl group or a haloalkyl group containing 1 to 12 carbon atoms and optionally terminated with a carboxyl group; and * denotes an asymmetric carbon atom.

2. A method according to claim 1, wherein the hydroxyester of formulae (1) and (2) is methyl 4-hydroxy-5-tetradecynoate.

3. A method according to Claim 1 or Claim 2, wherein the organic solvent is more than one of the solvents n-hexane, n-heptane, n-octane, isobutane, isopentane, isooctane, cyclopentane, cyclohexane, a halogen-containing hydrocarbon solvent (e.g. dichloromethane, trichloromethane and carbon tetrachloride), benzene, toluene, xylene, diethylether, diisopropylether, n-butylether, tetrahydrofuran and tetrahydropyran.

4. A method according to any preceding claim wherein the hydrolase is a lipase.

## Patentansprüche

1. Verfahren zur Herstellung eines optisch aktiven Hydroxyesters, welches die Schritte umfaßt:
(i) Zufügen einer Carbonsäure oder eines Carbonsäureanhydrids zu einer Mischung, die einen (R)-Hydroxyester der nachfolgenden Formel 1 und einen (S)-Hydroxyester der nachfolgenden Formel 2 umfaßt, in Gegenwart einer Hydrolase in einem organischen Lösungsmittel, um den optisch aktiven (R)-Hydroxyester zu einem optisch aktiven (R)-Diester der nachfolgenden Formel 3 selektiv zu acylieren, und
(ii) Abtrennen des optisch aktiven (S)-Hydroxyesters von dem optisch aktiven (R)-Diester: 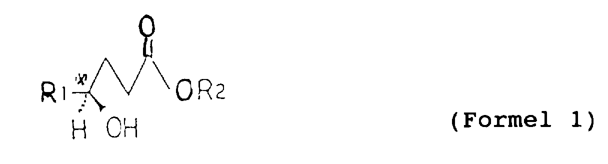 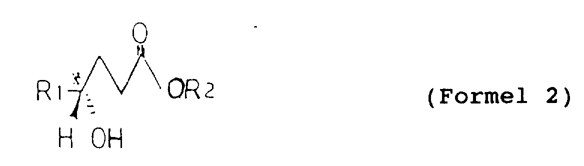 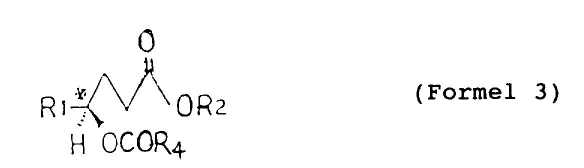
in denen R₁ -C≡C-R₃ oder -C=C-R₃ bedeuten; und R₂ und R₃ eine geradkettige oder verzweigte Alkylgruppe bedeuten, die 1 bis 10 Kohlenstoffatome enthält; R₄ bedeutet eine Alkylgruppe, eine Aralkylgruppe oder eine Haloalkylgruppe, die 1 bis 12 Kohlenstoffatome enthält und wahlweise mit einer Carboxylgruppe abgeschlossen ist; und * bedeutet ein asymmetrisches Kohlenstoffatom.

2. Verfahren nach Anspruch 1, dadurch gekennzeichnet, daß der Hydroxyester von Formeln (1) und (2) Methyl-4-hydroxy-5-tetradecinoat ist.

3. Verfahren nach Anspruch 1 oder Anspruch 2, dadurch gekennzeichnet, daß das organische Lösungsmittel mehr als eines der Lösungsmittel n-Hexan, n-Heptan, n-Oktan, Isobutan, Isopentan, Isooktan, Cyclopentan, Cyclohexan, ein halogenhaltiges Kohlenwasserstoff-Lösungsmittel (z. B. Dichlormethan, Trichlormethan und Kohlenstofftetrachlorid), Benzol, Toluol, Xylol, Diethylether, Diisopropylether, n-Butylether, Tetrahydrofuran und Tetrahydropyran ist.

4. Verfahren nach jedem der vorhergehenden Ansprüche, dadurch gekennzeichnet, daß die Hydrolase eine Lipase ist.

## Revendications

1. Procédé pour produire un ester hydroxylé optiquement actif comprenant les étapes :
(i) d'addition d'un acide carboxylique ou d'un anhydride carboxylique à un mélange comprenant un ester (R)-hydroxylé répondant à la formule 1 ci-dessous et un ester (S)-hydroxylé répondant à la formule 2 ci-dessous, en présence d'une hydrolase dans un solvant organique pour acyler sélectivement l'ester (R)-hydroxylé optiquement actif en un (R)-diester optiquement actif répondant à la formule 3 ci-dessous, et
(ii) de séparation de l'ester (S)-hydroxylé optiquement actif du (R)-diester optiquement actif : 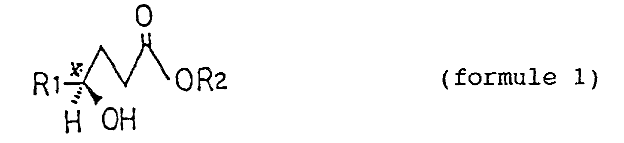 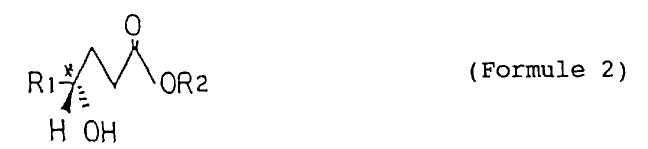 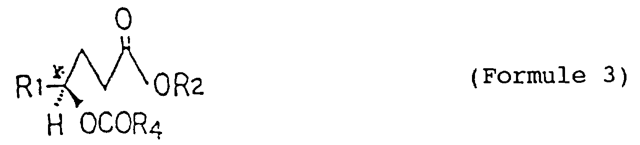
où R₁ représente -C≡C-R₃ ou -C=C-R₃; et R₂ et R₃ représentent un groupe alkyle à chaîne droite ou ramifié contenant 1 à 10 atomes de carbone, R₄ représente un groupe alkyle, un groupe aralkyle ou un groupe haloalkyle contenant 1 à 12 atomes de carbone et facultativement terminé par un groupe carboxyle; et * désigne un atome de carbone asymétrique.

2. Procédé selon la revendication 1, dans lequel l'ester hydroxylé répondant aux formules (1) et (2) est le 4-hydroxy-5-tétradécynoate de méthyle.

3. Procédé selon la revendication 1 ou 2, dans lequel le solvant organique est constitué de plus d'un des solvants suivants : le n-hexane, le n-heptane, le n-octane, l'isobutane, l'isopentane, l'isooctane, le cyclopentane, le cyclohexane, un solvant hydrocarboné contenant un halogène (par exemple le dichlorométhane, le trichlorométhane et le tétrachlorure de carbone), le benzène, le toluène, le xylène, l'éther diéthylique, l'éther diisopropylique, l'éther n-butylique, le tétrahydrofurane et le tétrahydropyrane.

4. Procédé selon l'une quelconque des revendications précédentes, dans lequel l'hydrolase est une lipase.
